# EUROPEAN PATENT APPLICATION

(11) **EP 3 248 672 A1**
(43) Date of publication of application: **29.11.2017**
(21) Application number: 16740172.8
(22) Date of filing: 19.01.2016
(51) Int. Cl.: B01D 71/68, B01D 69/00, B01D 69/08, B01D 71/44

(54) **POROUS HOLLOW FIBER FILTRATION MEMBRANE**

(30) Priority: 19.01.2015 JP 2015008058
(71) Applicant: Asahi Kasei Medical Co., Ltd., Chiyoda-ku Tokyo 101-8101 (JP)
(72) Inventor: KAYAMA, Yuzo, Tokyo 101-8101 (JP); KOMURO, Masayasu, Tokyo 101-8101 (JP); SHIRAISHI, Mitsugu, Tokyo 101-8101 (JP)
(74) Representative: dompatent von Kreisler Selting Werner - Partnerschaft von Patent- und Rechtsanwälten mbB
(86) International application number: PCT/JP2016/051458
(87) International publication number: WO 2016/117565

(57) **Abstract**

The present invention provides a porous hollow fiber filtration membrane comprising a polysulfone-based polymer and hydrophilic polymer, the porous hollow fiber filtration membrane including a gradient asymmetric structure in which the average pore diameter of fine pores increases from the outer surface toward the inner surface, a content of the hydrophilic polymer in the membrane is 6.0 to 12.0% by mass, and a ratio of the content of the hydrophilic polymer in the outer surface to the content of the hydrophilic polymer in the membrane is 1.20 to 1.60.

## Description

### Technical Field

The present invention relates to a porous hollow fiber filtration membrane.

### Background Art

In recent years, medical treatments using fractionated plasma products and biopharmaceuticals have been widespread because of their few side effects and high treatment effectiveness. However, the fractionated plasma products are derived from human blood, the biopharmaceuticals are derived from animal cells, and thus, there is a possibility that pathogenic substances such as viruses may is contaminated thereinto. If a fractionated plasma product or a biopharmaceuticals produced without including a process of treating pathogenic substances that may be contaminated thereinto is administered to a patient and the like, there is a risk of infection with a virus.

In processes for purifying a fractionated plasma product or a biopharmaceutical, a technique for adding safety against virus infection is used.

Examples of the technique for adding safety include methods for inactivating a virus and methods for removing a virus.

Examples of the method for inactivating a virus include heat treatment, optical treatment, and treatment with chemicals, and examples of the method for removing a virus include membrane filtration methods.

In recent years, due to problems of protein denaturation, inactivation efficiency, and contamination of chemicals in the methods for inactivating a virus, a membrane filtration method that is effective for any viruses irrespective of their thermal and chemical characteristics has been spread.

As types of viruses, there are small viruses, such as a parvovirus (18-24nm diameter) and a poliovirus (25-30nm diameter), and relatively large ones, such as an HIV virus (80-100nm diameter). In recent years, needs for removal of small viruses such as the parvovirus have been particularly increased.

The first performance required for a virus removal membrane used for the purification process is safety. The safety means not allowing pathogenic substances such as viruses to contaminate into fractionated plasma products and biopharmaceuticals.

The second performance required for the virus removal membrane is productivity. The productivity means recovering protein, such as albumin of 5 nm size or globulin of 10 nm size, efficiently.

From the viewpoint of the productivity, an ultrafiltration membrane and a hemodialysis membrane each having a pore diameter of around several nanometers, and a reverse osmosis membrane having a further smaller pore diameter are not suitable as a virus removal membrane because the protein blocks the pores during filtration, the flux is reduced during filtration, and the protein recovery rate is reduced. Moreover, when removal of a small virus such as a parvovirus is intended, an advanced technique to achieve both the safety and the productivity is required because the size of the virus and the size of protein are close to each other. Additionally, reduction in the flux during filtration is caused not only by blocking of pores due to a size factor but also by blocking of pores due to the adsorption of protein to the pore surface.

Accordingly, for virus removal membranes, not only the precise design of the pore diameter but also the design of the pore surface are important.

In Patent Literature 1, a virus removal membrane formed from a blend state of a polysulfone-based polymer and a copolymer of vinylpyrrolidone and vinyl acetate is disclosed, and that the copolymer of vinylpyrrolidone and vinyl acetate, which is a hydrophilic polymer, is homogeneously dispersed in the membrane thickness direction has been evaluated by measurement of the content of the hydrophilic polymer in the proximity to the outer surface and in the entire membrane.

In Patent Literature 2, there is disclosed a virus removal membrane obtained by coating a blend membrane of a polysulfone-based polymer and a copolymer of vinylpyrrolidone and vinyl acetate with a polysaccharide derivative.

### Citation List

### Patent Literature

Patent Literature 1: International Publication No. WO 2013/012024
Patent Literature 2: Japanese Patent No. 5403444

### Summary of Invention

### Technical Problem

In Patent Literature 1, the risk of protein adsorption to the membrane is at the same level in the entire membrane by homogeneously dispersing the hydrophilic polymer in the membrane thickness direction. However, in order to suppress the clogging due to adsorption of protein to the membrane during filtration of a protein solution, it is necessary to suppress adsorption of the protein in the dense layer, which is a region in which the pore diameters are the smallest in the membrane. However, this problem has not been solved.

In Patent Literature 2, the clogging due to adsorption of protein to the membrane during filtration of a protein solution is suppressed by coating a hollow fiber membrane formed in the same method as in Patent Literature 1 with a polysaccharide derivative after membrane-forming. From this, it is assumed that adsorption of protein to the membrane cannot be sufficiently suppressed in the intramembrane dispersion state of the hydrophilic polymer in a hollow fiber porous membrane formed by the method of Patent Literature 1. Moreover, when the membrane is coated after membrane-forming with a polysaccharide derivative, inhomogeneity in the dispersion state of the copolymer of vinylpyrrolidone and vinyl acetate is caused from the inhomogeneity in the dispersion state of the polysaccharide derivative on the pore surface. Thus the risk of protein adsorption to the pore surface does not reach the same level, and local protein adsorption propagates to the surroundings, leading to a risk of causing clogging. Then, in a membrane intended to filter a protein solution, it is necessary to suppress protein adsorption in the dense layer, but this problem has not been also solved.

The problem to be solved by the present invention is to provide a porous hollow fiber filtration membrane that suppresses adsorption of protein to the fine pore surface of a dense layer during filtration of a protein solution and has excellent protein permeability.

The present inventors have made extensive investigations to solve the above problem and, as a result, have found that the above problem is solved by controlling the content of the hydrophilic polymer in the outer surface and the membrane, thereby having completed the present invention.

That is, the present invention is as follows.
(1) A porous hollow fiber filtration membrane comprising:
   a polysulfone-based polymer; and
   a hydrophilic polymer, the porous hollow fiber filtration membrane including:
      a gradient asymmetric structure in which the average pore diameter of fine pores increases from the outer surface toward the inner surface;
      a content of the hydrophilic polymer in the membrane is 6.0 to 12.0% by mass; and
      a ratio of the content of the hydrophilic polymer in the outer surface to the content of the hydrophilic polymer in the membrane is 1.20 to 1.60.
(2) The porous hollow fiber filtration membrane according to (1), wherein a pure water permeation rate is 60 to 200 L/ (hr·m²·bar) .
(3) The porous hollow fiber filtration membrane according to (1) or (2), wherein a thickness of a dense layer is 2 to 10 µm.
(4) The porous hollow fiber filtration membrane according to any one of (1) to (3), wherein the polysulfone-based polymer is a polyethersulfone.
(5) The porous hollow fiber filtration membrane according to any one of (1) to (4), wherein the hydrophilic polymer is a copolymer comprising vinylpyrrolidone.
(6) The porous hollow fiber filtration membrane according to any one of (1) to (5), wherein the porous hollow fiber filtration membrane is used for removing a virus contained in a protein solution to collect the protein.

### Advantageous Effects of Invention

According to the present invention, it is possible to provide a porous hollow fiber filtration membrane that suppresses adsorption of protein to the fine pore surface during filtration and has excellent protein permeability.

### Brief Description of Drawings

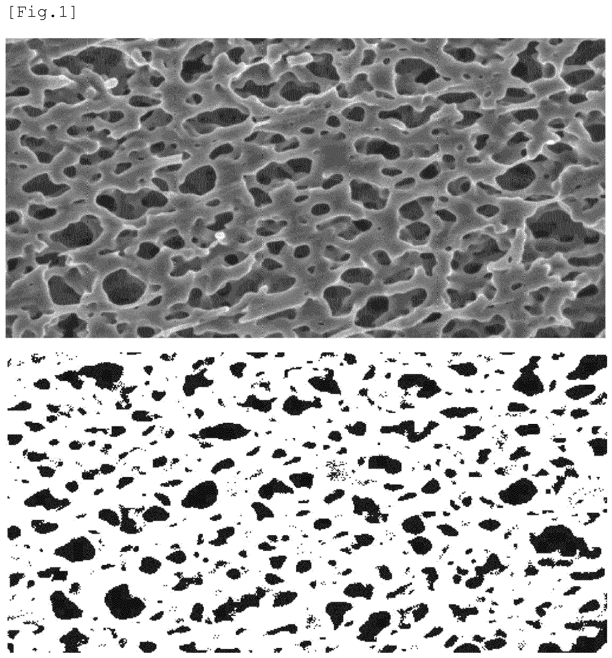

[Figure 1] Figure 1 shows an example of a result (Figure 1 below) obtained by binarizing an image observed with a scanning electron microscope into pore portions and solid portions (Figure 1 above). White portions represent solid portions and black portions represent pore portions.

### Description of Embodiments

Hereinafter, embodiments for carrying out the present invention (hereinafter, referred to as "present embodiments") will be described in detail. The present invention is not limited to the following embodiments, and various modifications of the embodiments can be carried out within the scope of the gist of the present invention.

The porous hollow fiber filtration membrane of the present embodiment contains a polysulfone-based polymer and a hydrophilic polymer and has a gradient asymmetric structure in which the average pore diameter of fine pores increases from the outer surface toward the inner surface. The content of the hydrophilic polymer in the membrane is 6.0 to 12.0% by mass, and the ratio of the content of the hydrophilic polymer in the outer surface to the content of the hydrophilic polymer in the membrane is 1.20 to 1.60.

The porous hollow fiber filtration membrane of the present embodiment contains a polysulfone-based polymer and a hydrophilic polymer.

Polysulfone-based polymers are polysulfones having a repeating unit represented by the formula 1 below, or polyether sulfones having a repeating unit represented by the formula 2 below.

Of polysulfone-based polymers, polyethersulfones are preferable from the viewpoint of solubility such as prevention of formation of associates in the solution.

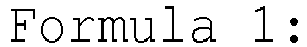

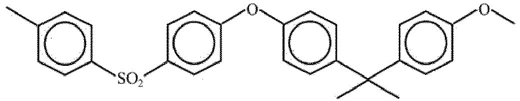

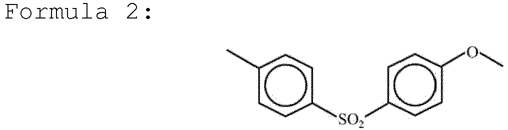

The polysulfone-based polymers may contain a substituent such as a functional group or an alkyl group, or a hydrogen atom in the hydrocarbon skeletons may be substituted by another atom such as a halogen or a substituent, in the structures represented by formula 1 and formula 2.

The polysulfone-based polymers may be used singly or two or more of these may be used in mixture.

The hydrophilic polymer is not particularly limited as long as the polymer is compatible with a polysulfone-based polymer, but copolymers containing vinylpyrrolidone are preferable in that such copolymers have a high compatibility with a polysulfone-based polymer and can mix a large amount of the hydrophilic polymer into a dope.

As the copolymer containing vinylpyrrolidone, copolymers of vinylpyrrolidone and vinyl acetate are preferable from the viewpoint of compatibility with the polysulfone-based polymer. It is preferable that the copolymerization ratio of vinylpyrrolidone to vinyl acetate be 6:4 to 9:1 from the viewpoint of adsorption of protein to the membrane surface and interaction with polysulfone-based polymers in the membrane.

Examples of the copolymer of vinylpyrrolidone and vinyl acetate specifically include LUVISKOL (trade name) VA 64 and VA 73, which are commercially available from

### BASF SE.

The hydrophilic polymers may be used singly or two or more of these may be used in mixture.

The porous hollow fiber filtration membrane of the present embodiment has a gradient asymmetric structure in which the average pore diameter of fine pores increases from the outer surface toward the inner surface.

In the present embodiment, the inner surface means the surface of the hollow portion side of the hollow fiber in the porous hollow fiber filtration membrane, and the outer surface means the membrane surface opposite to the hollow portion surface in the membrane thickness direction.

That the porous hollow fiber filtration membrane has a gradient asymmetric structure in which the average pore diameter of fine pores increases from the outer surface toward the inner surface is determined by photographing a hollow fiber cross section with a scanning electron microscope (SEM). For example, a visual field is set horizontally to the membrane thickness direction at an arbitrary portion of the hollow fiber cross section with a photographing magnification set to 50,000. After photographing the one visual field that is set, the photographing visual field is moved horizontally to the membrane thickness direction and the next visual filed is photographed. This photographing operation is repeated to take photographs of the cross section of the membrane without clearance, and the photographs thus obtained are connected to provide one photograph of the cross section of the membrane. In this cross-sectional photograph, the average pore diameter in a range of (2 µm in the circumferential direction of the membrane) × (1 µm from the outer surface toward the inner surface side) is calculated to digitize a gradient structure of the membrane cross section every micrometer from the outer surface toward the inner surface side.

By such digitalization, it is possible to confirm that the porous hollow fiber filtration membrane has a gradient porous structure in which the average pore diameter of fine pores increases from the outer surface toward the inner surface.

In the present embodiment, having a gradient asymmetric structure in which the average pore diameter of fine pores increases from the outer surface toward the inner surface may be a structure from which it can be confirmed that the average pore diameter tends to increase from the outer surface toward the inner surface, and does not mean that the average pore diameter in a range of 1 µm from the outer surface towards the inner surface side is the smallest and the average pore diameter in a range of 1 µm from the inner surface toward the outer surface side is the largest.

The average pore diameter is calculated by a method using image analysis. Specifically, pore portions and solid portions are subjected to binarization with Image-pro plus manufactured by Media Cyberbetics, Inc. The pore portions and the solid portions are discriminated based on brightness, and the sections that cannot be discriminated or noise is corrected with a free-hand tool. An edge section that forms a contour of a pore portion and a porous structure observed in the back of a pore portion are discriminated as a pore portion. After the binarization, the diameter of a pore is calculated from a value of an area per one pore assuming that the shape of the pore is a perfect circle. The calculation is conducted for every pore to calculate an average pore diameter every 1 µm × 2 µm range. It is to be noted that discontinuous pore portions at the ends of the visual fields are also counted.

In the present embodiment, a 1 µm × 2 µm range calculated to have an average pore diameter of 50 nm or less is defined as a dense layer.

When the fine pores in the dense layer are blocked, the flux is reduced. In other words, the flux of a membrane is dominated by the thickness of the dense layer and the pore diameter in the dense layer.

A membrane in which the average pore diameter of the fine pores decreases from the upstream side of filtration toward the downstream side of filtration can capture large particles in the upstream of filtration to thereby suppress the reduction in the flux due to blocking of the dense layer by large particles.

In the present embodiment, the porous hollow fiber filtration membrane has a gradient asymmetric structure in which the average pore diameter of fine pores increases from the outer surface toward the inner surface, and thus can suppress the reduction in the flux due to blocking of fine pores in the dense layer on the outer surface side.

There are two types of blocking of fine pores in the dense layer.

One type of blocking, in which fine pores smaller than the size of a solute are blocked by the solute, is caused due to size as a factor (size factor), and the other type of blocking, in which fine pores are blocked by adsorption of a solute to the fine pore surface, is caused due to adsorption as a factor (adsorption factor).

Blocking of fine pores due to the size factor occurs intermittently during filtration, blocking of fine pores due to the adsorption factor is often observed in the early stage of filtration.

In order to improve the protein permeability, it is important to suppress adsorption of protein to the fine pore surface in the dense layer, in addition to the size factor, to thereby suppress a marked reduction in the flux in the early stage of filtration.

The porous hollow fiber filtration membrane of the present embodiment has a content of the hydrophilic polymer in the membrane of 6.0 to 12.0% by mass, and a ratio of the content of the hydrophilic polymer in the outer surface to the content of the hydrophilic polymer in the membrane of 1.20 to 1.60.

In the present embodiment, by suppressing the amount of the hydrophilic polymer contained in the outer surface and the membrane, it is possible to suppress adsorption of protein to the fine pore surface in the dense layer to thereby suppress a reduction in the flux due to adsorption.

It is possible to suppress contraction of the fine pores due to the thickness of the hydrophilic polymer on the pore surface by setting the content of the hydrophilic polymer in the membrane to 6.0 to 12.0% by mass as the amount of the hydrophilic polymer contained in the outer surface and the membrane. Additionally, it is possible to suppress a reduction in the flux by setting the content of the hydrophilic polymer in the membrane to 6.0 to 12.0% by mass as the amount of the hydrophilic polymer contained in the outer surface and the membrane and setting the ratio of the content of the hydrophilic polymer in the outer surface to the content of the hydrophilic polymer in the membrane to 1.20 to 1.60.

In the present embodiment, measurement of the amount of the hydrophilic polymer contained in the outer surface and in the membrane is conducted as follows.

The porous hollow fiber filtration membrane is cut to a thickness of 10 µm in the membrane thickness direction. The cut porous hollow fiber filtration membrane piece is sandwiched with potassium bromide sheets. The sandwiched membrane, a section of 5 × 5 µm of which is set as one segment, is continuously measured by microscopic FT-IR using the transmission method in the membrane thickness direction. From the spectrum measured, a peak derived from the polysulfone-based polymer (around 1580 cm⁻¹) and a peak derived from the hydrophilic polymer (for example, around 1730 cm⁻¹ for a hydrophilic polymer having carbonyl bonds, and for example, around 1100 cm⁻¹ for hydrophilic polymer having ether bonds) are detected. The amount contained is calculated as the content of the hydrophilic polymer in each divided segment from the ratio of the absorbance area of the peak derived from the hydrophilic polymer/the absorbance area of the peak derived from the polysulfone-based polymer.

The average of the content of the hydrophilic polymer of each segment is used as the content of the hydrophilic polymer in the membrane.

The content of the hydrophilic polymer in the segment in the outermost surface side in the membrane thickness direction/the content of the hydrophilic polymer in the membrane is calculated as the ratio of the content of the hydrophilic polymer in the outer surface to the content of the hydrophilic polymer in the membrane.

In the present embodiment, it can be confirmed that, in the dense layer, a reduction in the flux due to adsorption of protein in the early stage of filtration is suppressed and excellent protein permeability is exhibited by a filtration test of immunoglobulin.

The filtration test of immunoglobulin can be conducted in accordance with the method described in Examples.

In the filtration test of immunoglobulin, that a reduction in the flux is suppressed means that the ratio (F₅/F_{w}) of the flux five minutes after filtration begins when 1.5% by mass of immunoglobulin is filtered at a constant pressure of 2.0 bar (F₅) to the value obtained by doubling the water permeability when pure water is filtered at a constant pressure of 1.0 bar (F_{w}) is 0.50 or more.

In order to increase the protein permeability, which is a useful component, it is preferable that operation can be conducted at a high filtration pressure, in addition that adsorption of protein is suppressed in the dense layer. It is possible to increase the flux by setting the filtration pressure to be high.

Operation at a high filtration pressure can be achieved by using a polysulfone-based polymer having pressure resistance as a base material.

In order to further increase the protein permeability, it is preferable to increase the permeation rate of pure water within a range in which virus-removing performance may be exerted. The permeation rate of pure water will be a guide for the flux, which is the filtration speed of a protein solution. Since the protein solution has a solution viscosity higher than the viscosity of pure water, the filtration rate of the protein solution becomes lower than the permeation rate of pure water. However, the higher the permeation rate of pure water, the higher the filtration rate of the protein solution becomes. The permeation rate of pure water of the porous hollow fiber filtration membrane of the present embodiment is preferably 60 to 200 L/ (hr·m²·bar) , more preferably 100 to 180 L/ (hr·m²·bar) .

When the permeation rate of pure water is 60 L/ (hr·m²·bar) or more, the filtration time is not too long, and protein can be collected with high efficiency. Additionally, when the permeation rate of pure water is 200 L/ (hr·m²·bar) or less, virus-removing performance can be exerted.

The permeation rate of pure water can be measured in accordance with the method described in Examples.

The virus-removing mechanism in a virus removal membrane is considered to be as follows.

A protein solution containing a virus permeates through a virus-removing layer in which a plurality of virus-capturing surfaces each being perpendicular to the permeation direction are stacked.

The distribution always exists in the size of the fine pores in the virus-capturing surfaces, and the virus is captured at fine pore portions of which size is smaller than that of the virus. Although the virus-capturing rate at one virus-capturing surface is low, high virus-removing performance is achieved by stacking a plurality of virus-capturing surfaces. For example, even if the virus-capturing rate at one virus-capturing surface is 20%, the whole virus-capturing rate becomes 99.999% by stacking 50 layers of the virus-capturing surfaces.

In a virus removal membrane, virus-capturing performance can be increased by capturing the virus with multiple layers, and thus, it is preferable to make the dense layer thick. From the viewpoint of suppressing a reduction in the flux of protein, it is preferable to set the thickness of the dense layer within a predetermined range.

From the viewpoint of achieving both the safety of virus removal and the productivity of the efficiency of protein recovery, the thickness of the dense layer is preferably 2 to 10 µm, more preferably 2 to 8 µm.

The parvovirus clearance of the porous hollow fiber filtration membrane of the present embodiment is preferably 4.0 or more, more preferably 5.0 or more, as an LRV.

From the similarity to the actual liquid as a parvovirus and easiness of operation, the parvovirus is preferably a porcine parvovirus (PPV).

The LRV of the PPV can be measure in accordance with the method described in Examples.

In the present embodiment, the porous hollow fiber filtration membrane is not particularly limited, and can be produced in the manner as in the following.

For example, a polysulfone-based polymer, a hydrophilic polymer, and a solvent are mixed until dissolved. The dissolved mixture is defoamed to provide a dope. The dope from the orifice of a tube-in-orifice type spinneret (spinneret) and an inner liquid (bore liquid) from the tube of the spinneret are each ejected simultaneously, pass through an air gap portion, and are introduced into a coagulation liquid to thereby form a hollow fiber. The hollow fiber obtained is wound after washing with water, is subjected to removal of liquid in the hollow portion and then heat treatment, and is dried.

As the solvent used in the dope, a wide range of solvents can be used as long as the solvent is a good solvent for the polysulfone-based polymer and the hydrophilic polymer as well as the solvent is compatible with the polysulfone-based polymer and the hydrophilic polymer. Examples include N-methyl-2-pyrrolidone (NMP), N,N-dimethylformamide (DMF), N-N-dimethylacetoamide (DMAc), dimethyl sulfoxide, and ε-caprolactam. Amide-based solvents such as NMP, DMF, and DMAc are preferable, and NMP is more preferable.

To the dope, a non-solvent is preferably added. Examples of the non-solvent used in the dope include glycerin, water, and diol compounds, and the diol compounds are preferable.

The diol compound is a compound having a hydroxy group at both ends of the molecule, and as the diol compound, a compound represented by the following formula 3 and having an ethylene glycol structure of which number of repeating unit n is 1 or more, is preferable.

Examples of the diol compound include diethylene glycol (DEG), triethylene glycol (TriEG), tetraethylene glycol (TetraEG), and polyethylene glycols. DEG, TriEG, and TetraEG are preferable, and TriEG is more preferable.

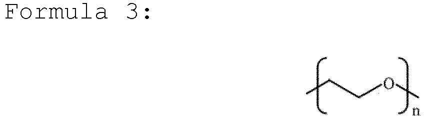

The mass ratio of solvent/non-solvent in the dope may be in approximately the same amount and is preferably 35/65 to 65/35. When the amount of the non-solvent is 65/35 or less as the mass ratio, coagulation progresses at an appropriate rate, and an excessively large pore diameter is unlikely to occur. Thus, it is possible to obtain a porous hollow fiber filtration membrane having a membrane structure preferable as a filtration membrane for protein-solution treatment applications. The amount of the non-solvent of 35/65 or more based on a mass ratio is preferable because progress of coagulation is not too fast, an excessively small pore diameter is unlikely to occur, and furthermore, macrovoids, which lead to structure defects, are unlikely to occur.

The concentration of the polysulfone-based polymer in the dope is preferably 15 to 35% by mass, more preferably 20 to 30% by mass.

By setting the concentration of the polysulfone-based polymer in the dope to 15% by mass or more, it is possible to achieve an appropriate membrane strength. Meanwhile, by increasing the porosity, it is possible to obtain high permeation performance. By setting the concentration of the polysulfone-based polymer in the dope to 35% by mass or less, the porosity does not become too low, the permeation performance can be maintained, and additionally, the virus capture volume of the membrane can be kept high.

The concentration of the hydrophilic polymer in the dope is preferably 5 to 12% by mass.

By setting the concentration of the hydrophilic polymer in the dope to 5% by mass or more, the membrane to be obtained will be sufficiently hydrophilized. Even in use for filtration of a protein solution, the concentration is preferably 5% by mass or more, from the viewpoint that the protein is unlikely to be adsorbed to the membrane and a reduction in the flux is unlikely to occur. Setting the concentration of the hydrophilic polymer of the dope to 12% by mass or less is preferable because the thickness of the hydrophilic polymer on the pore surface is not too large in the membrane to be obtained and the pore diameter does not become excessively small. Additionally, from the viewpoint of preventing fiber sticking, wherein hollow fibers are bonded to one another after drying, the concentration is preferably 12% by mass or less.

The dope is obtained by dissolving a polysulfone-based polymer, a hydrophilic polymer, a solvent, and a non-solvent under stirring at a constant temperature. The temperature at this time is preferably 30 to 60°C, which is higher than normal temperature. Tertiary or lower nitrogen-containing compounds (NMP, vinylpyrrolidone, and the like) are oxidized in air, and the oxidation further easily progresses when the compounds are warmed. Thus, preparation of the dope is preferably conducted under an inert gas atmosphere. Examples of the inert gas include nitrogen and argon, and nitrogen is preferable from the viewpoint of production costs.

If bubbles exist in the dope, large bubbles become responsible for fiber breakage during spinning and small bubbles form macrovoids after membrane formation, which become responsible for structure defects of the membrane. Thus, degassing is preferably conducted.

A degassing process can be conducted as follows.

The inside of a tank containing a completely dissolved dope is warmed to 50°C, decompressed to 2 kPa, and is left to stand for 1 hour or more. This operation is preferably repeated 7 times or more. The pressure in degassing is preferably higher than the boiling point of the solvent. In order to improve the deforming efficiency, the dope may be stirred during degassing.

Foreign matter is removed from dope preferably before ejection from the spinneret. Large foreign matter becomes responsible for fiber breakage during spinning, and small foreign matter becomes responsible for structure defects of the membrane. By using raw materials containing little foreign matter, risks of mixing of foreign matter can be reduced.

In order to remove mixing of foreign matter from the packing of the dope tank and the like, a filter is preferably installed before the dope is ejected from the spinneret, and filters of different pore diameters are more preferably installed in multiple stages. Specifically, it is preferable that a mesh filter having a pore diameter of 10 µm and a mesh filter having a pore diameter of 3 µm be installed in this order from the position nearer to the dope tank.

As the inner liquid used in membrane forming, it is preferable to use components identical to those used in the dope and coagulation liquid. When NMP/TriEG, for example, are used as the solvent/non-solvent for the dope and NMP/TriEG and water are used as the solvent/non-solvent of the coagulation liquid, the inner liquid is preferably constituted from NMP/TriEG and water.

When the amount of the solvents in the inner liquid is increased, there is an effect of delaying the progress of coagulation and allowing the membrane structure formation to progress slowly. When the amount of the non-solvent is increased, there is an effect of delaying the diffusion of the solution, delaying the progress of coagulation and allowing the membrane structure formation to progress slowly due to a thickening effect. When water is increased, there is an effect of accelerating the progress of coagulation.

In order to facilitate the progress of coagulation appropriately to control the membrane structure, thereby obtaining a preferable membrane structure for the porous hollow fiber filtration membrane of the present embodiment, it is preferable that the ratio of the solvent/non-solvent, which are the organic components in the inner liquid, is in almost the same amount, for example, 35/65 to 65/35 based on a mass ratio and the ratio of the organic components/water is 70/30 to 100/0 based on a mass ratio.

The temperature of the spinneret is preferably 40 to 60°C to obtain an appropriate pore diameter.

After being ejected from the spinneret, the dope passes through the air gap portion and is introduced into the coagulation bath. The retention time on the air gap portion is preferably 0.01 to 0.75 seconds, more preferably 0.05 to 0.4 seconds. By setting the retention time to 0.01 seconds or more, coagulation before introduction into the coagulation bath is allowed to progress sufficiently to thereby achieve an appropriate pore diameter. By setting the detention time to 0.75 seconds or less, excessive progress of coagulation is prevented to enable the membrane structure in the coagulation bath to be precisely controlled.

A draft ratio is preferably 1.1 to 6.0, more preferably 1.1 to 4.0 in order to control drawing to a hollow fiber membrane in the spinning process. A draft ratio means a ratio of a take-over rate to a linear rate of ejection of the dope from the spinneret.

A high draft ratio means that the draw ratio after the dope is ejected from the spinneret is high.

Generally, in forming a membrane by a wet phase separation method, most of the membrane structure is determined when a dope passes through an air gap portion and exits from the coagulation bath. The inside of the membrane is configured of solid portions formed by entanglement of polymer chains and hollow portions, which are pore portions where no polymer exists. The detailed mechanism is not clear, but when the hollow fiber membrane is excessively drawn before coagulation is completed, in other words, when the membrane is excessively drawn before the polymer chains become entangled, the entanglements of polymer chains are torn off. Thus, pore portions are connected, which leads to formation of excessively large pores, or pore portions are divided, which leads to formation of excessively small pores. The excessively large pores become a cause of leakage of the virus, and the excessively small pores become a cause of clogging. Even few structure defects become fatal to a membrane intended for virus removal, and thus, the draft ratio is preferably made small as much as possible.

The dope passes through the filter and the spinneret, is moderately coagulated in the air gap portion, and is thereafter introduced into the coagulation liquid. Of the hydrophilic polymer in the dope introduced into the coagulation liquid, a portion is involved in coagulation of the polysulfone-based polymer to be captured into the membrane, and the other portion, which is not involved in coagulation of the polysulfone-based polymer, diffuses into the coagulation liquid. By adding the hydrophilic polymer in the coagulation liquid to thereby make the gradient between the concentration of the hydrophilic polymer in dope and the concentration of the hydrophilic polymer in the coagulation liquid milder, this diffusion rate is delayed, and the content of the hydrophilic polymer in the outer surface side of the hollow fiber membrane higher than the average in the entire membrane is achieved. It has been found that the amount of the hydrophilic polymer to be added to the coagulation liquid is 2.0 to 7.0% by mass in the coagulation liquid in order to set the ratio of the content of the hydrophilic polymer in the outer surface to the content of the hydrophilic polymer in the entire membrane to 1.20 to 1.60.

The coagulation liquid is constituted from a solvent, a non-solvent, water, and a hydrophilic polymer. The solvent in the coagulation liquid has an effect of delaying coagulation, and water has an effect of accelerating coagulation. Thus, in the composition of the coagulation liquid, it is preferable that the mass ratio of the solvent/non-solvent be 35/65 to 65/35 and the mass ratio of the solvent/water be 70/30 to 10/90. The preferable upper limit of the mass ratio of solvent/water is set from the viewpoint of allowing coagulation to progress appropriately to thereby prevent formation of excessively large pores. The preferable lower limit is set from the viewpoint of preventing formation of excessively small pores due to excessively rapid coagulation.

The temperature of the coagulation bath is preferably 30 to 60°C from the viewpoint of pore diameter control.

The spinning rate is not particularly limited as long as the rate is the condition under which a porous hollow fiber filtration membrane having no defect can be obtained, and is preferably 5 to 15 m/min. The spinning rate is preferably 5 m/min or more from the viewpoint of productivity, and preferably 15 m/min or less from the viewpoint of ensuring the retention time in the air gap portion and enabling the draft ratio to be proper.

The hollow fiber membrane pulled up from the coagulation bath is introduced into a water washing tank and washed with warm water. When the air retention time until the hollow fiber membrane pulled up from the coagulation bath is introduced into the water washing tank is extremely increased, drying of the hollow fibers from the outer surface side excessively progresses during air retention. Since the hydrophilic polymer exists in the coagulation bath, the hydrophilic polymer contained in the coagulation bath exists in the proximity to the outer surface of the hollow fiber membrane which has exited from the coagulation bath. In order to suppress excessive drying of outer surface side of the porous fibers and efficiently wash off the excess hydrophilic polymer in the proximity to the outer surface, the retention time in air from the coagulation bath to the water washing tank is preferably less than 10 seconds.

In the washing process with warm water, it is preferable to make sure to remove the solvent and the hydrophilic polymer which is not fixed to the membrane without fault. When the hollow fiber membrane is dried while containing a solvent, the solvent is concentrated in the membrane during drying and a polysulfone-based polymer is dissolved or swollen, leading to a possibility of changing the membrane structure. When the hydrophilic polymer which is not fixed to the membrane remains, the pores are blocked, leading to possibility of reducing the permeability of the membrane. In order to increase the diffusion rate of the solvent and non-solvent to be removed and the hydrophilic polymer which is not fixed to the membrane and increase the washing efficiency with water, it is preferable that the temperature of the warm water be 50°C or higher. In the water washing process, a Nelson roller is preferably used. In order to conduct water washing sufficiently, the retention time of the hollow fiber membrane in the bath for washing with water is preferably 80 seconds or more from the viewpoint of preventing change into an unpreferable membrane structure as a virus removal membrane due to the remaining NMP. The water washing process, which is intended to remove unnecessary components, is preferably as long as possible, but, in respect of production efficiency, the duration of the process is appropriately 300 seconds or less.

The hollow fiber membrane pulled up from the bath for washing with water is wound to a reel with a winder. At this point, if the hollow fiber membrane is wound in air, the membrane gradually becomes dried and contracts although slightly. Then, the contraction degrees of the membrane in the initial stage and in the latter stage differ, and thus, the membrane structures differ. This becomes responsible for the inhomogeneity of the hollow fiber membrane obtained in the production process. Accordingly, the hollow fiber membrane is preferably wound in water.

Both ends of the porous fiber membrane wound to the reel are cut, and the membrane is then made into a bundle and is allowed to be held by a support not to loosen. Then, the hollow fiber membrane held is immersed and washed in hot water. In the hollow portion of the hollow fiber membrane wound to the reel, a white turbid liquid remains in which polysulfone-based polymer fine particles having a size of nanometers to micrometers are suspended. When the hollow fiber membrane is dried without removing the white turbid liquid, the fine particles of the polysulfone-based polymer may block the pores of the hollow fiber membrane, leading to a reduction in the membrane performance. Thus, the white turbid liquid in the hollow portion is preferably removed.

In the hot-water treatment process, the hollow fiber membrane is also washed from the inner surface side, and thus, the hydrophilic polymer which has not been fixed to the membrane and the like which have not been removed in the washing process with water are efficiently removed. The temperature of the hot water is preferably 50 to 100°C. Setting the temperature of the hot water to 50°C or more is preferable because the washing efficiency can be increased. The washing time is preferably 30 to 120 minutes. The hot water is preferably exchanged several times during washing.

In the present embodiment, the wound hollow fiber membrane is preferably high-pressure hot water treated. Specifically, it is preferable that the hollow fiber membrane, in a state where the membrane is completely immersed in water, is placed in a high-pressure steam sterilizer and treated at 120°C or more for 2 to 6 hours. The detailed mechanism is not clear, but it is conceived that, by this high-pressure hot-water treatment, not only the solvent and non-solvent slightly remaining in the hollow fiber membrane and the hydrophilic polymer which is not fixed to the membrane are completely removed but also the state of existence of the polysulfone-based polymer and hydrophilic polymer are optimized leading to optimization of the structure preferable as the porous hollow fiber filtration membrane in the present embodiment.

The porous hollow fiber filtration membrane of the present embodiment is obtained by drying such as air drying, drying under reduced pressure, or hot-air drying. Without particular limitation, the hollow fiber membrane is preferably dried in a state where both ends thereof are fixed such that the membrane does not contract during drying.

### Examples

Hereinafter, the present embodiment will be described in detail with Examples, but the present invention is not limited to Examples below. The measurement method in Examples is as follows.

### (1) Measurement of Inner Diameter and Membrane Thickness

The inner diameter of the porous hollow fiber filtration membrane was determined by photographing the vertical cross section of the membrane with a stereoscopic microscope.

The outer diameter was determined in the same manner as the inner diameter, and the membrane thickness was calculated from (Outer diameter - Inner diameter) / 2.

Additionally, the membrane area was calculated from Inner diameter × π × Effective length of porous hollow fiber filtration membrane.

### (2) Measurement of Pure-Water Permeation rate

The amount of pure water of 25°C filtered was measured by dead-end filtration at a constant pressure of 1.0 bar, and the water permeability was measured from the filtration time and used as the pure-water permeation rate.

### (3) Filtration Test of Immunoglobulin

A filter assembled such that the number of the porous hollow fiber filtration membrane was four and the effective length was 8 cm was subjected to high-pressure sterilization treatment at 122°C for 60 minutes. A solution was prepared using Venoglobulin IH 5% I.V. (2.5 g/50 ml) (manufactured by Mitsubishi Tanabe Pharma Corporation) such that the solution has an immunoglobulin concentration of 1.5% by mass, a sodium chloride concentration of 0.1 M, and a pH of 4.5. The prepared solution was subjected to dead-end filtration at a constant pressure of 2.0 bar. The integrated amount filtered five minutes after filtration began / 5 was taken as F₅, and F_{5/}F_{w} was calculated from the water permeability measured in (2).

### (4) Measurement of Porcine Parvovirus Clearance

A solution obtained by spiking 0.5% by volume of a PPV solution to the solution prepared in (3) Filtration Test of Immunoglobulin was used as a filtration solution. A filtration test was conducted as in (3) Filtration Test of Immunoglobulin. The titer (TCID₅₀ value) of the filtrate was measured by a virus assay. The virus clearance of the PPV was calculated from LRV = Log (TCID₅₀) / mL (filtration solution) - Log (TCID₅₀) / mL (filtrate) .

### (5) Measurement of Dense Layer

The cross section of the hollow fiber membrane was photographed with a scanning electron microscope (SEM) by setting a visual field horizontally to the membrane thickness direction of the cross section of the hollow fiber with a photographing magnification of 50,000. After photographing the one visual field that was set, the photographing visual field was moved horizontally to the membrane thickness direction and the next visual filed was photographed. This photographing operation was repeated to take photographs of the cross section of the membrane without clearance, and the photographs thus obtained were connected to provide one photograph of the cross section of the membrane. In this cross-sectional photograph, the average pore diameter in a range of (2 µm in the circumferential direction of the membrane) × (1 µm from the outer surface toward the inner surface side) was calculated to digitize an inclination structure of the membrane cross section every micrometer from the outer surface toward the inner surface side. The average pore diameter was calculated in the manner as described below. Pore portions and solid portions were subjected to binarization with Image-pro plus manufactured by Media Cybernetics, Inc. in such a way that the pore portions and the solid portions were discriminated based on brightness, the sections that were not able to be discriminated or noise was corrected with a free-hand tool, and an edge section forming a contour of a pore portion and a porous structure observed in the back of a pore portion were discriminated as a pore portion. After the binarization, the diameter of a pore was calculated from a value of an area per one pore assuming that the shape of the pore was a perfect circle. Discontinuous pore portions at the ends of the visual fields were also counted, and the calculation was conducted for every pore to calculate an average pore diameter every 2 µm × 1 µm range. A range where the average pore diameter was 50 nm or smaller was defined as a dense layer, and a range where the average pore diameter was larger than 50 nm was defined as a coarse layer.

### (6) Thickness of Dense Layer

The thickness of a dense layer was measured as "the number of ranges showing an average pore diameter of 50 nm or smaller × 1 µm".

### (7) Measurement of Content of Hydrophilic Polymer in Membrane and Ratio of Content of Hydrophilic Polymer in Outer Surface to Content of Hydrophilic Polymer in Membrane

The porous hollow fiber filtration membrane was cut to a thickness of 10 µm in the membrane thickness direction. The cut porous hollow fiber filtration membrane piece is sandwiched with potassium bromide sheets and was formed into a tablet using a pressing machine. The tablet formed, a section of 5 × 5 µm of which is set as one segment, was continuously measured by microscopic FT-IR using the transmission method in the membrane thickness direction. From the spectrum measured, a peak derived from the polysulfone-based polymer and a peak derived from the hydrophilic polymer were detected. The amount contained was calculated as the content of the hydrophilic polymer in each divided segment from the ratio of the absorbance area of the peak derived from the hydrophilic polymer/the absorbance area of the peak derived from the polysulfone-based polymer.

The average of the content of the hydrophilic polymer of each segment was taken as the content of the hydrophilic polymer in the membrane.

The content of the hydrophilic polymer in the segment in the outermost surface side in the membrane thickness direction/the content of the hydrophilic polymer in the membrane was calculated as the ratio of the content of the hydrophilic polymer in the outer surface to the content of the hydrophilic polymer in the membrane.

### (Example 1)

A solution obtained by mixing 27 parts by mass of a polyethersulfone(PES) (Ultrason (trade name) E6020P manufactured by BASF SE), 9 parts by mass of a copolymer of vinylpyrrolidone and vinyl acetate (Luviskol (R) VA64 manufactured by BASF SE, hereinbelow referred to as "VA64"), 30.4 parts by mass of NMP (manufactured by Kishida Chemical Co., Ltd.), and 33.6 parts by mass of TriEG (manufactured by Kanto Chemical Co., Inc.) at 50°C and then repeating degassing under a reduced pressure 7 times was used as a dope. The dope was ejected from a orificeof a tube-in-orifice type spinneret with the temperature of a spinneret set to 50°C, and a mixed liquid of 45.1 parts by mass of NMP, 49.9 parts by mass of TriEG, and 5 parts by mass of water was ejected as an inner liquid from a tube.

The ejected dope and inner liquid passed through an air gap portion and were allowed to run in a coagulation liquid comprising 26.8 parts by mass of NMP, 29.6 parts by mass of TriEG, 37.6 parts by mass of water, and 6 parts by mass of VA64 at 30°C. The hollow fiber membrane pulled out from the coagulation bath was allowed to run in a water washing tank set at a temperature of 55°C by a Nelson roller, and then wound in water. The spinning rate was set to 8 m/min, and the draft ratio was set to 2. The wound hollow fiber membrane was cut at both ends, made into a bundle and held by a support not to loosen. The membrane was then immersed in hot water of 80°C and washed for 60 minutes. The washed hollow fiber membrane was subjected to high-pressure hot-water treatment under the condition of 128°C for six hours and dried in a vacuum to thereby provide a porous hollow fiber filtration membrane.

### (Example 2)

A porous hollow fiber filtration membrane was obtained in the same manner as in Example 1 except that the coagulation bath composition was changed to 27.4 parts by mass of NMP, 30.2 parts by mass of TriEG, 38.4 parts by mass of water, and 4 parts by mass of VA64.

### (Example 3)

A porous hollow fiber filtration membrane was obtained in the same manner as in Example 1 except that the coagulation bath composition was changed to 28.0 parts by mass of NMP, 30.8 parts by mass of TriEG, 39.2 parts by mass of water, and 2 parts by mass of VA64.

### (Example 4)

A porous hollow fiber filtration membrane was obtained in the same manner as in Example 1 except that the coagulation bath composition was changed to 18.6 parts by mass of NMP, 20.6 parts by mass of TriEG, 58.8 parts by mass of water, and 2 parts by mass of VA64.

### (Example 5)

A porous hollow fiber filtration membrane was obtained in the same manner as in Example 1 except that the coagulation bath composition was changed to 9.3 parts by mass of NMP, 10.3 parts by mass of TriEG, 78.4 parts by mass of water, and 2 parts by mass of VA64.

### (Example 6)

A porous hollow fiber filtration membrane was obtained in the same manner as in Example 5 except that the composition of the dope was changed to 26 parts by mass of PES, 10 parts by mass of VA64, 32 parts by mass of NMP, and 32 parts by mass of TriEG.

### (Example 7)

A porous hollow fiber filtration membrane was obtained in the same manner as in Example 1 except that the composition of the dope was changed to 24 parts by mass of PES, 12 parts by mass of VA64, 30.4 parts by mass of NMP, and 33.6 parts by mass of TriEG and the coagulation liquid composition was changed to 37.3 parts by mass of NMP, 29.9 parts by mass of TriEG, 28.8 parts by mass of water, and 4 parts by mass of VA64.

### (Example 8)

A porous hollow fiber filtration membrane was obtained in the same manner as in Example 1 except that the composition of the dope was changed to 30 parts by mass of PES, 6 parts by mass of VA64, 30.4 parts by mass of NMP, and 33.6 parts by mass of TriEG and the coagulation liquid composition was changed to 24.8 parts by mass of NMP, 27.4 parts by mass of TriEG, 42.8 parts by mass of water, and 5 parts by mass of VA64.

Measurement results of the porous hollow fiber filtration membranes obtained in Examples 1 to 8 in respect to (1) to (7) are shown in Table 1.

All the porous hollow fiber filtration membranes obtained in Examples 1 to 8 suppressed adsorption of protein to the fine pore surface during filtration and had excellent protein permeability.

### (Comparative Example 1)

A hollow fiber membrane was obtained in the same manner as in Example 1 except that the coagulation liquid composition was changed to 26.2 parts by mass of NMP, 29.0 parts by mass of TriEG, 36.8 parts by mass of water, and 8 parts by mass of VA64.

Measurement results of the hollow fiber membrane obtained in Comparative Example 1 in respect to (1) to (7) are shown in Table 1. The hollow fiber membrane obtained in Comparative Example 1 had a higher content of the hydrophilic polymer in the outer surface and did not exhibit excellent protein permeability.

### (Comparative Example 2)

A hollow fiber membrane was obtained in the same manner as in Example 1 except that the coagulation liquid composition was changed to 28.5 parts by mass of NMP, 31.5 parts by mass of TriEG, and 40.0 parts by mass of water.

Measurement results of the hollow fiber membrane obtained in Comparative Example 2 in respect to (1) to (7) are shown in Table 1. The hollow fiber membrane obtained in Comparative Example 2 could not suppress adsorption of protein in the dense layer and did not exhibit excellent protein permeability.

### (Comparative Example 3)

A hollow fiber membrane was obtained in the same manner as in Example 7 except that the coagulation liquid composition was changed to 30.9 parts by mass of NMP, 34.2 parts by mass of TriEG, 27.9 parts by mass of water, and 7 parts by mass of VA64.

Measurement results of the hollow fiber membrane obtained in Comparative Example 3 in respect to (1) to (7) are shown in Table 1. The hollow fiber membrane obtained in Comparative Example 3 had a higher content of the hydrophilic polymer and a reduced pore diameter in the dense layer, and thus, did not exhibit excellent protein permeability.

### (Comparative Example 4)

A hollow fiber membrane was obtained in the same manner as in Example 8 except that the coagulation liquid composition was changed to 25.6 parts by mass of NMP, 28.3 parts by mass of TriEG, 44.1 parts by mass of water, and 2 parts by mass of VA64.

Measurement results of the hollow fiber membrane obtained in Comparative Example 4 in respect to (1) to (7) are shown in Table 1. The hollow fiber membrane obtained in Comparative Example 4 had a lower content of the hydrophilic polymer, could not suppress adsorption of protein in the dense layer, and did not exhibit excellent protein permeability.

**[Table 1]**

| | Example 1 | Example 2 | Example 3 | Example 4 | Example 5 | Example 6 | Example 7 | Example 8 | Comparative Example 1 | Comparative Example 2 | Comparative Example 3 | Comparative Example 4 |
|---|---|---|---|---|---|---|---|---|---|---|---|---|
| Inner diameter | 201 | 200 | 203 | 199 | 200 | 201 | 203 | 198 | 200 | 202 | 198 | 200 |
| Membrane thickness | 60 | 59 | 61 | 60 | 60 | 61 | 62 | 58 | 60 | 61 | 60 | 59 |
| Content of hydrophilic polymer in membrane | 9.9 | 9.2 | 8.6 | 8.8 | 9.3 | 10.3 | 11.8 | 6.4 | 10.5 | 7.8 | 12.7 | 5.7 |
| Ratio of contents of hydrophilic polymer (in outer surface site/in membrane) | 1.52 | 1.38 | 1.25 | 1.27 | 1.29 | 1.25 | 1.22 | 1.56 | 1.70 | 1.07 | 1.56 | 1.29 |
| Thickness of dense layer | 5 | 4 | 4 | 2 | 1 | 1 | 4 | 2 | 5 | 4 | 5 | 2 |
| Pure water permeation rate | 109 | 158 | 179 | 214 | 232 | 195 | 170 | 86 | 64 | 190 | 89 | 103 |
| F₅/F_{w} | 0.60 | 0.57 | 0.53 | 0.51 | 0.51 | 0.51 | 0.57 | 0.55 | 0.31 | 0.45 | 0.34 | 0.42 |
| LRV | 5 | 4.5 | 4.5 | 3 | 2 | 3.5 | 3.5 | 3.5 | - | 4 | - | 2.5 |

The present application is based on the Japanese Patent Application No. 2015-8058 filed with the Japan Patent Office on January 19, 2015, the content of which is incorporated herein by reference.

### Industrial Applicability

The porous hollow fiber filtration membrane in the present invention has industrial applicability in purification processes of fractionated plasma products and biopharmaceuticals. The porous hollow fiber filtration membrane can be used for removing a virus contained in a protein solution to collect the protein.

## Claims

1. A porous hollow fiber filtration membrane comprising:
a polysulfone-based polymer; and
a hydrophilic polymer, the porous hollow fiber filtration membrane including:
a gradient asymmetric structure in which the average pore diameter of fine pores increases from the outer surface toward the inner surface;
a content of the hydrophilic polymer in the membrane is 6.0 to 12.0% by mass; and
a ratio of the content of the hydrophilic polymer in the outer surface to the content of the hydrophilic polymer in the membrane is 1.20 to 1.60.

2. The porous hollow fiber filtration membrane according to claim 1, wherein a pure water permeation rate is 60 to 200 L/ (hr·m²·bar) .

3. The porous hollow fiber filtration membrane according to claim 1 or 2, wherein a thickness of a dense layer is 2 to 10 µm.

4. The porous hollow fiber filtration membrane according to any one of claims 1 to 3, wherein the polysulfone-based polymer is a polyethersulfone.

5. The porous hollow fiber filtration membrane according to any one of claims 1 to 4, wherein the hydrophilic polymer is a copolymer comprising vinylpyrrolidone.

6. The porous hollow fiber filtration membrane according to any one of claims 1 to 5, wherein the porous hollow fiber filtration membrane is used for removing a virus contained in a protein solution to collect the protein.
